# EUROPEAN PATENT SPECIFICATION

(11) **EP 0 607 064 B1**
(45) Date of publication and mention of the grant of the patent: **05.04.2000**
(21) Application number: 94400026.4
(22) Date of filing: 05.01.1994
(51) Int. Cl.: C12Q 1/32, G01N 33/84

(54) **Assay method and reagent for magnesium in human body fluids**
Testverfahren und Reagenz für Magnesium in menschlichen Körperflüssigkeiten
Méthode d'essai et réactif pour magnésium dans des liquides du corps humain

(30) Priority: 14.01.1993 JP 2083793
(43) Date of publication of application: 20.07.1994
(73) Proprietor: ORIENTAL YEAST CO., LTD., Tokyo (JP)
(72) Inventor: Kawakami, Yukari, Izumiohtu-shi, Osaka-fu (JP); Fujita, Tsuyoshi, Ikeda-shi, Osaka-fu (JP)
(74) Representative: Clisci, Serge

(56) References cited:
- EP-A- 0 207 493
- EP-A- 0 371 600
- US-A- 4 657 854
- CLINICAL CHEMISTRY. vol. 32, no. 4 , April 1986 , WINSTON US pages 629 - 632 MICHAEL C. WIMMER, JOSEPH D. ARTISS, AND BENNIE ZAK 'A Kinetic Colorimetric Procedure for Quantifying Magnesium in Serum'

## Description

### Field of the Invention

Magnesium is commonly found in most animal and plant cells, and magnesium ion is indispensable for growth and the sustaining of life.

Also, magnesium in the body fluctuates in a competitive manner with calcium, and since such fluctuation has been observed in cases of dysfunction of the central nervous system or the heart, or in cases of kidney failure, acute pancreatitis, etc., magnesium assays are becoming important objects for examination.

An assay method for magnesium according to the present invention makes possible a quick, accurate assay of magnesium in human blood serum, and thus provides a great contribution to clinical examinations.

### Description and Disadvantages of the Prior Art

In the past, known methods for the measurement of magnesium have included the colorimetric method wherein a chelate is produced using xylidyl blue, and the atomic absorption method, etc., but of these methods the former has disadvantages from the point of view of specificity, while the latter has a weakness in that special equipment is required.

Furthermore, a method has been proposed wherein a reaction solution containing isocitrate dehydrogenase (hereunder referred to as iCDH), NADP+ and isocitrate is used in Reaction formula (I) shown in the following Table 1, using Mg++ to start the reaction, and the amount of the resulting NADPH is measured by the absorbance at 340 nm (USP 4,657,854).

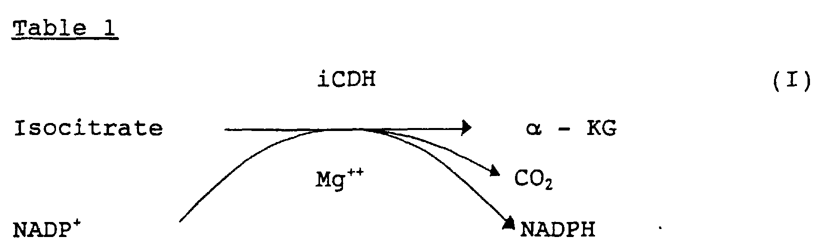

However, when assays of Mg++ in human blood serum have been made according to this method, it has not been possible to obtain accurate values.

The reason is that, even at very low amounts, Mg++ reacts strongly and terminates the reaction according to formula (I), and therefore an accurate calibration line has been unobtainable, and it has been impossible to know the exact Mg++ content.

In published JP-A-2/145 196 and corresponding EP-A-0 371 600 of the Applicant a method is disclosed wherein a reaction solution containing iCDH, NADP⁺, isocitrate and a Mg⁺⁺ chelating agent is added to a solution containing Mg⁺⁺, the mixture is allowed to stand for a while, and then the increase in the amount of NADPH is measured.

However, even in this method, it has been impossible to obtain a straight line for the increase in the amount of NADPH with the absorbance at 340 nm, and an accurate measurement of the Mg⁺⁺ content has been difficult.

### Subject Matter of the Invention

The present invention was accomplished for the purpose of an accurate assay of the Mg⁺⁺ content in untreated or treated body fluids.

### Brief Description of the Drawings

Fig. 1 shows normal values for a Mg⁺⁺ assay of 0-10 mg/dl according to Example 1 (absorbance at 340 nm).
Fig. 2 shows normal values for a Mg⁺⁺ assay of 0-50 mg/dl according to Example 1 (absorbance at 340 nm).

### Detailed Description of the Invention

According to the present invention, it is possible to make an accurate assay of the Mg⁺⁺ content in untreated or treated body fluids, by using two separate assay reagents 1 and 2, with assay reagent 1 containing no iCDH, but containing a Mg⁺⁺ chelating agent, and assay reagent 2 containing iCDH and NADP⁺, and using both successively at a predetermined interval.

That is, almost all of the Mg⁺⁺ in the untreated or treated body fluid is chelated by the addition of the assay reagent 1 which contains no iCDH but contains a Mg++ chelating agent, and after a determined period of time passes a very minute amount of Mg++ remains, and this very minute amount of Mg++ is measured after adding the assay reagent 2 which contains iCDH and NADP+, based on the activation of iCDH by Mg++ and on the accompanying increase in NADPH determined by the increase in the absorbance at 340 nm.

Compared to the method disclosed in JP-A-2/145196 and corresponding EP-A-0 371 600 wherein the measurement is made using a reagent which contains a Mg⁺⁺ chelating agent, iCDH and NADP⁺, the method according to the present invention wherein the assay reagent 1 which contains no ICDH but contains a Mg⁺⁺ chelating agent and the assay reagent 2 which contains iCDH and NADP⁺ are used separately, is superior for the assaying of Mg⁺⁺ in body fluids, and the measured values of Mg⁺⁺ according to the present invention are extremely accurate.

The fluid to be assayed containing Mg⁺⁺ may be blood serum, urine, or the like, and the measurement is made with the undiluted original solution.

According to this invention is also provided a combined assay reagent for assaying magnesium in human body fluids, comprising:
an assay reagent 1 containing a Mg⁺⁺ chelating agent and isocitrate ; and,
an assay reagent 2 containing isocitrate dehydrogenase and NADP⁺.

The assay reagent 1 may be composed by dissolving a Mg⁺⁺ chelating agent, a Mn⁺⁺ chelating agent and potassium isocitrate in a Tris buffer solution.

The assay reagent 2 may be composed by dissolving iCDH, NADP⁺ and potassium phosphate in water.

The reaction may be conducted under heated conditions. The assay reagent 1 is added to the untreated or treated body fluid, which is heated to about 37°C, and the Mg⁺⁺ is chelated for about 5 minutes. The standing time is the period during which almost all of the Mg⁺⁺ has been chelated, and since the content of Mg⁺⁺ differs depending on the sample, it is necessary to preestablish a standing time for each sample which will produce an accurate straight line during the following reaction corresponding to the amount of Mg⁺⁺.

After the solution is allowed to stand for the determined period of time, the assay reagent 2 is added thereto, and under heated conditions of about 37°C the increase in the amount of NADPH from 2 to 3 minutes after the addition is measured based on the increase in the absorbance at 340 nm, and when this value is applied to the reference line the Mg⁺⁺ content in the original body fluid may be accurately known.

The Mg⁺⁺ chelating agent to be used according to the present invention may be any of the following substances or their salts. EDTA, trans-1,2-cyclohexanediamine-N,N,N',N'-tetraacetic acid, N,N-di(hydroxyethyl)glycine, 1,3-diaminopropane-2-ol-N,N,N',N'-tetraacetic acid, diethylenetriamine-N,N,N',N'',N'''-pentaacetic acid, ethylenediamine-N,N'-diacetic acid, ethylenediamine-N,N'-propionic acid, N-hydroxyethylethylenediamine-N,N',N'-triacetic acid, ethylenediamine-N,N,N',N'-tetrakis(methylenephosphonic acid), glycol ether diamine-N,N,N',N'-tetraacetic acid, hexamethylenediamine-N,N,N',N'-tetraacetic acid, hydroxyethyliminodiacetic acid, iminodiacetic acid, 1,2-diaminopropane-N,N,N',N'-tetraacetic acid, nitrilotriacetic acid, nitrilotripropionic acid, nitrilotris(methylenephosphonic acid), triethylenetetraamine-N,N,N',N'',N''',N'''-hexaacetic acid, ethylenediamine-N,N'-bis(methylenephosphonic acid), ethylenediamine di(o-hydroxyphenylacetic acid), 1,2-bis(2-aminophenoxy)ethane-N,N,N',N'-tetraacetic acid.

Also, the iCDH is activated by manganese ion, but it is possible to avoid the influence of the manganese ion by including in the reaction solvent a chelating agent, for example GEDTA, which selectively traps manganese ion without trapping magnesium ion.

According to the present invention, most of the Mg⁺⁺ is deactivated by the Mg⁺⁺ chelating agent which contains no iCDH, and the property whereby the activity of iCDH increases precisely depending on the remaining minute amount of Mg⁺⁺ is used for a specific, quick, highly sensitive assay of magnesium.

By the method according to the present invention, it is possible to make an accurate assay of magnesium using a minute amount of a specimen.

The Examples according to the present invention will now be provided.

### Example 1

In 10 ml of purified water were dissolved 42.3 mg of MgCl₂.H₂O, to prepare a standard solution of 250 mg/dl of Mg⁺⁺. Said standard solution was diluted to prepare solutions of 0, 2, 4, 6, 8, 10, 20, 30, 40 and 50 mg/dl, respectively, of Mg⁺⁺.

To 5 µl of each of these solutions were added 240 µl of the assay reagent 1 shown in Table 2, and the mixture was allowed to stand at 37°C for 5 minutes, after which 60 µl of the assay reagent 2 shown in Table 3 were added thereto.

**Table 2**

| Assay reagent 1 | |
|---|---|
| EDTA | 12.5 mM |
| GEDTA | 5 mM |
| Potassium isocitrate | 6.25 mM |
| Tris buffer solution | 100 ml |
| (pH 9.0) | |

**Table 3**

| Assay reagent 2 | |
|---|---|
| NADP+ | 10 mM |
| iCDH | 25 u/ml |
| Potassium phosphate | 10 mM |
| Water | 100 ml |
| (pH 7.0) | |

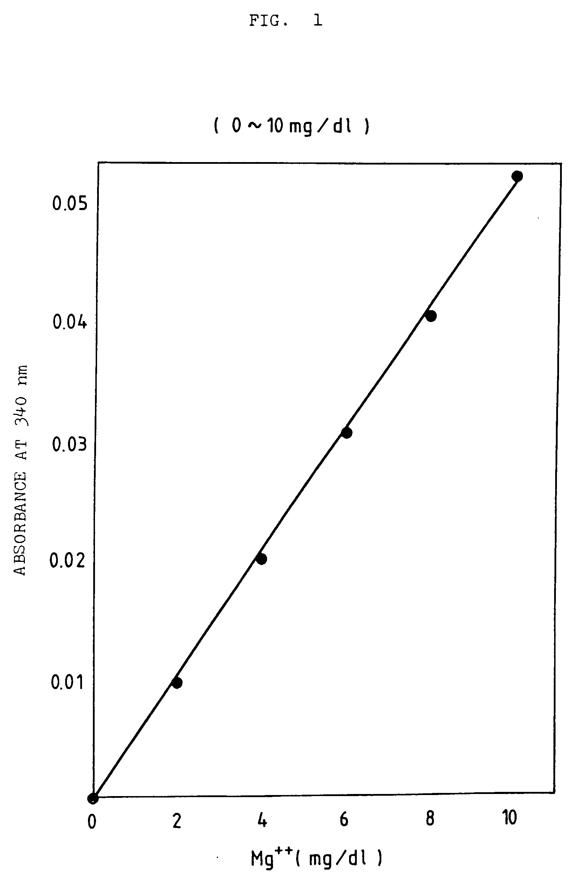

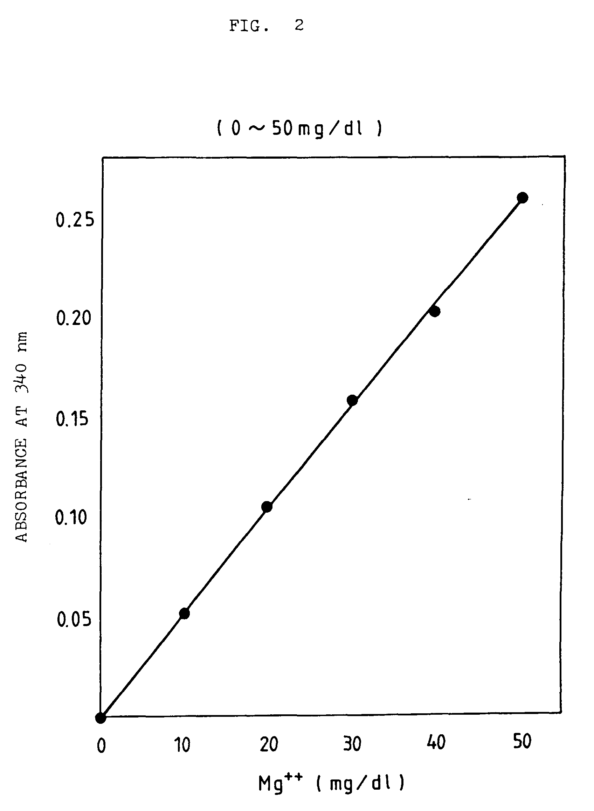

The change in the absorbance at 340 nm from 2 to 3 minutes after addition of the assay reagent 2 was measured by rate assay. The results are shown in Figs. 1 and 2, and they were established as reference lines.

### Example 2

Serum was separated from healthy human blood, 240 µl of the assay reagent 1 shown in Example 1 were added to 5 µl of the separated serum, and the mixture was allowed to stand at 37°C for 5 minutes, after which 60 µl of the assay reagent 2 shown in Example 1 were added thereto and the mixture was allowed to stand at 37°C for 2 minutes, the increase in the amount of absorbance at 340 nm during 2-3 minutes was measured, and rate assay was made, whereby a value of 2.5 mg Mg⁺⁺/100 ml serum was obtained.

### Example 3

To 5 µl of healthy human urine were added 240 µl of the assay reagent 1 shown in Example 1, and the mixture was allowed to stand at 37°C for 5 minutes, after which 60 µl of the assay reagent 2 shown in Example 1 were added thereto, the mixture was allowed to stand at 37°C for 2 minutes, the increase in the amount of absorbance at 340 nm during 2-3 minutes was measured, and rate assay was made, whereby a value of 8.0 mg Mg⁺⁺/100 ml urine was obtained.

## Claims

1. An assay method for magnesium in human body fluids, characterized by adding an assay reagent 1 which contains no isocitrate dehydrogenase but contains a Mg⁺⁺ chelating agent, to an untreated or treated body fluid, allowing the mixture to stand for a determined period of time to cause almost all of the Mg⁺⁺ to bond to the Mg⁺⁺ chelating agent, and thereafter adding an assay reagent 2 which contains isocitrate dehydrogenase and NADP⁺, converting the NADP⁺ into NADPH with the remaining Mg⁺⁺, and measuring the amount of Mg⁺⁺ in the human body fluid based on the increase in the amount of NADPH.

2. An assay method for magnesium in human body fluids, characterized by measuring the increase in the amount of NADPH according to claim 1 by the increase in the absorbance at 340 nm.

3. A combined assay reagent for assaying magnesium in human body fluids comprising :
an assay reagent 1 containing a Mg⁺⁺ chelating agent and isocitrate ; and,
an assay reagent 2 containing isocitrate dehydrogenase and NADP⁺.

4. A combined assay reagent according to claim 3, wherein said assay reagent 1 further contains a Mn⁺⁺ chelating agent.

5. A combined assay reagent according to any one of claims 3 and 4, wherein said assay reagent 2 further contains potassium phosphate.

6. An assay method according to any one of claims 1 and 2, wherein said assay reagent 1 further contains isocitrate.

7. An assay method according to claim 6, wherein said isocitrate is potassium isocitrate.

8. An assay method according to claim 1 for magnesium in human body fluids, characterized by adding an assay reagent 1 which contains no isocitrate dehydrogenase but contains a Mg⁺⁺ chelating agent, a Mn⁺⁺ chelating agent and potassiuum isocitrate, to an untreated or treated body fluid, allowing the mixture to stand for a determined period of time to cause almost all of the Mg⁺⁺ to bond to the Mg⁺⁺ chelating agent, and thereafter adding an assay reagent 2 which contains isocitrate dehydrogenase, NADP⁺ and potassium phosphate, converting the NADP⁺ into NADPH with the remaining Mg⁺⁺, and measuring the amount of Mg⁺⁺ in the human body fluid based on the increase in the amount of NADPH.

9. A combined assay reagent according to any one of claims 3 and 5, wherein said isocitrate is potassium isocitrate.

## Patentansprüche

1. Assay-Verfahren für Magnesium in menschlichen Körperflüssigkeiten, **gekennzeichnet durch**
die Zugabe eines Assay-Reagenz 1, welches keine Isocitratdehydrogenase, aber einen Mg⁺⁺-Chelatbildner enthält, zu einer unbehandelten oder behandelten Körperflüssigkeit, Stehenlassen der Mischung für einen vorherbestimmten Zeitraum, um beinahe das gesamte des Mg⁺⁺ an den Mg⁺⁺-Chelatbildner zu binden, und anschließende Zugabe eines Assay-Reagenz 2, welches Isocitratdehydrogenase und NADP⁺ enthält, Umwandeln des NADP⁺ in NADPH mit dem verbleibenden Mg⁺⁺ und Messen der Menge an Mg⁺⁺ in der menschlichen Körperflüssigkeit bezogen auf die NADPH-Erhöhung.

2. Assay-Verfahren für Magnesium in menschlichen Körperflüssigkeiten, **gekennzeichnet durch**
das Messen der Erhöhung der Menge an NADPH gemäß Anspruch 1 durch die Erhöhung in der Absorption bei 340 nm.

3. Kombiniertes Assay-Reagenz zum Bestimmen von Magnesium in menschlichen Körperflüssigkeiten enthaltend
ein einen Mg⁺⁺-Chelatbildner und Isocitrat enthaltendes Assay-Reagenz 1 und
ein Isocitratdehydrogenase und NADP⁺ enthaltendes Assay-Reagenz 2.

4. Kombiniertes Assay-Reagenz nach Anspruch 3, worin das Assay-Reagenz weiter einen Mn⁺⁺-Chelatbildner enthält.

5. Kombiniertes Assay-Reagenz nach einem der Ansprüche 3 und 4, worin das Assay-Reagenz weiter Kaliumphosphat enthält.

6. Assay-Verfahren nach einem der Ansprüche 1 und 2, worin das Assay-Reagenz 1 weiter Isocitrat enthält.

7. Assay-Verfahren nach Anspruch 6, worin das Isocitrat Kaliumisocitrat ist.

8. Assay-Verfahren für Magnesium in menschlichen Körperflüssigkeiten nach Anspruch 1,
**gekennzeichnet durch**
die Zugabe eines Assay-Reagenz 1, welches keine Isocitratdehydrogenase, aber einen Mg⁺⁺-Chelatbildner, einen Mn⁺⁺-Chelatbildner und Kaliumisocitrat enthält, zu einer behandelten oder unbehandelten Körperflüssigkeit, Stehenlassen der Mischung über einen vorherbestimmten Zeitraum, um beinahe das gesamte Mg⁺⁺ an den Mg⁺⁺-Chelatbildner zu binden, und anschließende Zugabe eines Assay-Reagenz 2, welches Isocitratdehydrogenase, NADP⁺ und Kaliumphosphat enthält, Umwandlung des NADP⁺ in NADPH mit dem verbleibenden Mg⁺⁺ und Messen der Menge an Mg⁺⁺ in der menschlichen Körperflüssigkeit bezogen auf die Erhöhung in der Menge an NADPH.

9. Kombiniertes Assay-Reagenz nach einem der Ansprüche 3 und 5, worin das Isocitrat Kaliumcitrat ist.

## Revendications

1. Méthode de dosage du magnésium dans les liquides organiques humains, caractérisée par l'ajout d'un réactif de dosage 1, qui ne contient pas d'isocitrate déshydrogénase, mais contient un agent chélatant de Mg⁺⁺, à un liquide organique humain non traité ou traité, l'attente pendant une durée déterminée afin que tous les ions Mg⁺⁺ se lient à l'agent chélatant de Mg⁺⁺, puis l'ajout d'un réactif de dosage 2, qui contient de l'isocitrate déshydrogénase et du NADP⁺, conversion du NADP⁺ en NADPH avec le Mg⁺⁺ restant, et mesure de la quantité de Mg⁺⁺ dans le liquide organique humain basée sur l'augmentation de la quantité de NADPH.

2. Méthode de dosage du magnésium dans les liquides organiques humains, caractérisée par la mesure de l'augmentation de la quantité de NADPH selon la revendication 1 à partir de l'augmentation de l'absorbance à 340 nm.

3. Réactif de dosage combiné pour le dosage du magnésium dans les liquides organiques humains comprenant :
un réactif de dosage 1 contenant un agent chélatant de Mg⁺⁺ et de l'isocitrate ; et
un réactif de dosage 2 contenant de l'isocitrate déshydrogénase et du NADP⁺.

4. Réactif de dosage combiné selon la revendication 3, dans lequel ledit réactif de dosage 1 contient également un agent chélatant de Mn⁺⁺.

5. Réactif de dosage combiné selon l'une quelconque des revendications 3 et 4, dans lequel ledit réactif de dosage 2 contient également du phosphate de potassium.

6. Méthode de dosage selon l'une quelconque des revendications 1 et 2, dans laquelle ledit réactif de dosage 1 contient également de l'isocitrate.

7. Méthode de dosage selon la revendication 6, dans laquelle ledit isocitrate est de l'isocitrate de potassium.

8. Méthode de dosage selon la revendication 1 du magnésium dans les liquides organiques humains, caractérisée par l'ajout d'un réactif de dosage 1, qui ne contient pas d'isocitrate déshydrogénase, mais contient un agent chélatant de Mg⁺⁺, un agent chélatant de Mn⁺⁺ et de l'isocitrate de potassium, à un liquide organique humain non traité ou traité, l'attente pendant une durée déterminée afin que presque tous les ions Mg⁺⁺ se lient à l'agent chélatant de Mg⁺⁺, puis l'ajout d'un réactif de dosage 2, qui contient de l'isocitrate déshydrogénase, du NADP⁺, et du phosphate de potassium, conversion du NADP⁺ en NADPH avec le Mg⁺⁺ restant, et mesure de la quantité de Mg⁺⁺ dans le liquide organique humain basée sur l'augmentation de la quantité de NADPH.

9. Réactif de dosage combiné selon l'une quelconque des revendications 3 et 5, dans lequel ledit isocitrate est de l'isocitrate de potassium.
